Europäisches Patentamt

European Patent Office

Office européen des brevets

(19)

(11) Publication number : **0 483 086 A2**

(12) **EUROPEAN PATENT APPLICATION**

(21) Application number : **91830456.9**

(22) Date of filing : **25.10.91**

(51) Int. Cl.$^5$ : **A61K 37/02, A61K 47/48**

(30) Priority : **26.10.90 IT 4841590**

(43) Date of publication of application :
**29.04.92 Bulletin 92/18**

(84) Designated Contracting States :
**AT BE CH DE DK ES FR GB GR LI LU NL SE**

(71) Applicant : **FONDAZIONE ANDREA CESALPINO**
**169 Via Sicilia**
**I-00187 Roma RM (IT)**

(72) Inventor : **Balsano, Francesco**
**44 Via Clitunno**
**I-00198 Roma RM (IT)**
Inventor : **Barnaba, Vincenzo**
**86 Via Mascagni**
**I-00199 Roma RM (IT)**

(74) Representative : **Di Cerbo, Mario et al**
**Società Italiana Brevetti S.p.A., Piazza di Pietra 39**
**I-00186 Roma (IT)**

(54) **Process for the preparation of drugs for the treatment of hepatitis B, and drugs so obtained.**

(57)     A process for the preparation of drugs for the treatment of hepatitis B, characterized by the identification of transferrin receptor CD71 as a membrane structure responsible for the HBV infection. The drugs according to the invention for the treatment of hepatitis B can be characterized by the fact of comprising as active agent the soluble transferrin receptor bound to a toxin, for example the tetanus toxin. The invention relates also to drugs for the treatment of hepatitis B, characterized by the fact of comprising as active agent peptides homologous to amino acid sequence of HBsAg envelope protein of HBV. Hence, these peptides act as competitors with the virus for the entry into the cells through transferrin receptor CD71.

EP 0 483 086 A2

The present invention refers to a process for the preparation of drugs for the treatment of hepatitis B and relates also to drugs so obtainable. The invention is novel and involves an inventive step, since, having regard to the state of the art, it is not obvious to a person skilled in the art. The invention is based on the discovery of the mechanism by which hepatitis B virus, HBV, enters into the cells.

As is known, the most recent therapy of hepatitis B is based on the use of alpha-interferons and beta-interferons. These substances have proved to decrease HBV replication but not to eliminate it. In confirmation thereof, it must be pointed out that quite a number of patients treated with the above intereferons do not benefit from this therapy treatment. Therefore, more efficient treatments are needed in this specific field, as proposed by the present invention.

As is known, HBV replicates only in hepatocytes, even though it can gain access to practically any cell. It is also known that in humans T lymphocytes never present soluble antigens unless they possess a membrane receptor with a molecular structure capable to bind the antigen itself.

It has now been surprisingly found that T lymphocytes can process lipoprotein HBsAg (Hepatitis B surface Antigen) of HBV envelope and present fragments of antigen bound to class II MHC (Major Histocompatibility Complex) molecules to class II-restricted cytotoxic T lymphocytes that in turn kill the antigen presenting T cells. This means that T lymphocytes possess on their surface a molecular receptor-like structure capable to bind HBV.

Unexpectedly, it has been found that the receptor of such HBsAg lipoprotein of HBV is homologous to transferrin receptor CD71. This receptor is expressed not only on activated T cells, but also on all activated cells, including hepatocytes. Transferrin receptor CD71 so plays a crucial role in cell infection by HBV.

The subject matter of the present invention is therefore a process for the preparation of drugs for the treatment of hepatitis B, characterized by the identification of transferrin receptor CD71 as a membrane structure responsible for the infection by HBV.

The drugs according to the invention for the treatment of hepatitis B can be characterized by the fact of comprising as active agent the soluble transferrin receptor of CD71 bound to a toxin, for example the tetanus toxin. The invention relates also to drugs for the treatment of hepatitis B characterized by the fact of comprising as active agent peptides homologus to amino acid sequence of HBsAg envelope protein of HBV. These peptides act competing with the virus for the entry into the cells through transferrin receptor CD71.

The drugs according to the invention can also comprise as active agents the soluble transferrin receptor CD71 bound to a toxin and peptides homologous to the amino acid sequence of HBsAg envelope protein of HBV.

As previously said, the present invention is based on the discovery of the mechanism by which hepatitis B virus, HBV, infects the cells. Therefore, it could be interesting to summarize the experiments that have led to the finding of HBsAg receptor. Firstly, HBsAg specific T cell clones have been generated from hepatitis B vaccine recipients and their ability has been demonstrated to specifically kill other non-specific T clones, used as HBsAg-presenting cells. After that, using monoclonal antibodies specific for epitopes of different membrane structures, T cell receptors have been blocked one by one to inhibit HBsAg uptake by presenting T clones and to consequently inhibit its presentation to specific T clones.

The monoclonal antibody specific for transferrin receptor CD71 has been the sole monoclonal antibody (MAB) capable to inhibit HBsAg uptake by T cells, blocking specific cytolysis when added before but not after the antigen pulsing. Although also monoclonal antibodies directed against adhesion molecules (anti-CD2, anti-LFA3, anti-LFA1 and anti-ICAM1) have been able to block the specific cytolysis, their effect has been to inhibit the adhesive interactions between the T cells and not the initial uptake of HBsAg. As a matter of fact, in contrast with MABs anti-CD71, these MABs have maintained their inhibitory effect when added either before or after the pulsing.

In order to confirm the role of transferrin receptor CD71, further experiments have been performed, directed to block the HBsAg entry into the T cells. That is, soluble transferrin has been used, which has shown capable to down-regulate CD71 receptor and thereafter to block the virus entry into che cells.

## Claims

1. Process for the preparation of drugs for the treatment of hepatitis B, characterized by the identification of transferrin receptor CD71 as a membrane structure responsible for the HBV infection.

2. Drugs for the treatment of hepatitis B, characterized by the fact of comprising as active agent the soluble transferrin receptor CD71 bound to a toxin.

3. Drugs for the therapy of hepatitis B as per claim 2, in which the soluble transferrin receptor CD71 is bound to tetanus toxin.

4. Drugs for the treatment of hepatitis B, characterized by the fact of comprising as active agent peptides homologous to amino acid sequence of HBsAg envelope protein of HBV virus, which act as competitors with the virus for the entry into the cells through transferrin receptor CD71.

5.  Drugs for the treatment of hepatitis B, characterized by the fact of comprising as active agents the soluble transferrin receptor CD71 bound to a toxin and peptides homologous to amino acid sequence of HBsAg envelope protein of HBV virus.

6.  Drugs for hepatitis B therapy, as per claim 5, in which the soluble transferrin receptor CD71 is bound to tetanus toxin.